# EUROPEAN PATENT APPLICATION

(11) **EP 3 085 366 A1**
(43) Date of publication of application: **26.10.2016**
(21) Application number: 15164724.5
(22) Date of filing: 22.04.2015
(51) Int. Cl.: A61K 31/135, A61P 25/14

(54) **NMDA ANTAGONISTS FOR THE TREATMENT OF MENTAL DISORDERS WITH OCCURRENCE OF AGGRESSIVE AND/OR IMPULSIVE BEHAVIOR**

(71) Applicant: Institut du Cerveau et de la Moelle Épinière-ICM, 75013 Paris (FR); Assistance Publique-Hôpitaux de Paris (APHP), 75001 Paris (FR); Centre National de la Recherche Scientifique (C.N.R.S.), 75016 Paris (FR); Institut National de la Santé et de la Recherche Médicale (INSERM), 75654 Paris Cedex 13 (FR); Université Paris 6 Pierre et Marie Curie UPMC, 75005 Paris (FR)
(72) Inventor: Pouget, Pierre, 75015 Paris (FR); Missal, Marcus, 1180 Bruxelles (BE)
(74) Representative: Icosa

(57) **Abstract**

The present invention relates to the use of N-Methyl-D-aspartate (NMDA) antagonists at sub-anesthetic doses for the treatment of mental disorders with occurrence of aggressive and/or impulsive behavior.

## Description

### FIELD OF INVENTION

The present invention relates to the use of N-Methyl-D-aspartate (NMDA) antagonists at sub-anesthetic doses for the treatment of mental disorders with occurrence of aggressive and/or impulsive behavior.

### BACKGROUND OF INVENTION

Physicians tend to treat mental disorders with occurrence of aggressive and/or impulsive behavior with psychiatric medication from different groups. Antipsychotics are also used for borderline personality. Stimulants are notably used for attention deficit hyperactivity disorder (ADHD). Drugs such as selective serotonin reuptake inhibitors (SSRIs) can help both depression and impulsivity. Anticonvulsant drugs can help reduce impulsive, angry outbursts. Other drugs such as risperidone (Risperidal) have been helpful with both depression and feelings of depersonalization in people with borderline personality. Antidepressants or anxiolytics (including sedatives) are used for the treatment of anxiety disorders. Mood stabilizers are used primarily in bipolar disorder.

However, these groups of drug can induce major problems with adverse side effects of medication, tolerance and addiction in a patient. Alternative therapies aiming at decreasing side effects, tolerance effect, and toxicity effect are thus needed.

*N*-Methyl-D-aspartate receptor system is involved in numerous physiological and pathological processes in the central nervous system. Indeed, NMDA receptor overactivation is often associated with mental disorders with occurrence of aggressive and/or impulsive behavior. Therefore inhibition of NMDA receptors has often been considered as a good target to treat these disorders. However, blocking NMDA receptors has demonstrated the enhancement of impulsivity (Malhorta et al Neuropsychopharmacology. 1997 Sep; 17(3):141-50). In addition, the administration of ketamine in healthy humans and animal models induces schizophrenia (Breier et al Am J Psychiatry. 1997 Jun;154(6):805-11.). In particular, most previous studies have shown that ketamine injection increased impulsivity in animals and humans (Cottone et al. Psychopharmacology (Berl). 2013 Mar; 226(1):127-38).

Contrary to the state of the art, the inventors of the present application discovered that the administration of a low and sub-anesthetic dose of an NMDA receptor antagonist could restore attention, suppress and/or decrease impulsivity and/or aggressiveness in primates. Contrary to rodents, these species constitute a good model to study mental disorders with occurrence of aggressive and/or impulsive behavior since their prefrontal cortex is well developed and organized on the same plan as in humans.

### SUMMARY

One object of the invention is a composition for use in the treatment of a mental disorder with occurrence of aggressive and/or impulsive behavior in a subject in need thereof, comprising a sub-anesthetic dose of a compound antagonizing NMDA receptors.

In one embodiment, said compound is a noncompetitive or an uncompetitive antagonist of the NMDA receptor.

In another embodiment, said compound is ketamine, amantadine, tiletamine, phencyclidine (PCP), dizocilpine MK-801, Argiotoxin 636, dextrorphan, HU-211, Rhynchophylline, memantine, aptiganel, ifenprodil, lanicemine, Remacemide and/or analog and/or functional derivatives thereof.

In another embodiment, said compound is ketamine and/or an analog and/or a functional derivative thereof.

In another embodiment, said compound is memantine and/or an analog and/or a functional derivative thereof.

In another embodiment, said composition further comprises at least one pharmaceutical excipient.

In another embodiment, said composition is to be administered in a subject in need thereof by topical, transdermal, intramuscular, subcutaneous, oral, parenteral, intranasal administration.

In another embodiment, said composition is provided in a sustained-release form.

In another embodiment, said composition is formulated as a patch.

In another embodiment, said disorder comprises: personality disorders, anxiety disorders, mood disorders, impulse control and addiction disorders, attention deficit hyperactivity disorder (ADHD), obsessive-compulsive disorder (OCD), Tourette syndrome, Impulse control disorder (ICD), Blepharospasm, Tic disorder, Dissocial personality disorder, Intermittent explosive disorder (IED), Bipolar disorder, Pyromania, Kleptomania, paranoid personality disorders, schizoid personality disorders, schizotypal personality disorders, social anxiety disorder, panic disorder, agoraphobia, post-traumatic stress disorder, antisocial personality disorders, borderline personality disorders, histrionic personality disorders, narcissistic personality disorders.

Another object of the invention is a patch for use in the treatment of a mental disorder with occurrence of aggressive and/or impulsive behavior, comprising the composition of the invention, and an acceptable carrier.

### DEFINITIONS

In the present invention, the following terms have the following meanings:
- **"Anticipatory saccades"** or **"premature saccades"** refer to eye movements that are initiated before the appearance of an expected visual stimulus. Orienting to a newly appealing target by means of a saccadic eye movement is a very natural response. Also if the time and place of a target's appearance can be predicted, an anticipatory saccade often occurs before the target itself appears, or too briefly subsequently for visual guidance to have occurred. They can be qualified as premature or anticipatory if the consequence of anticipation is negative for the subject, like the absence of reward.

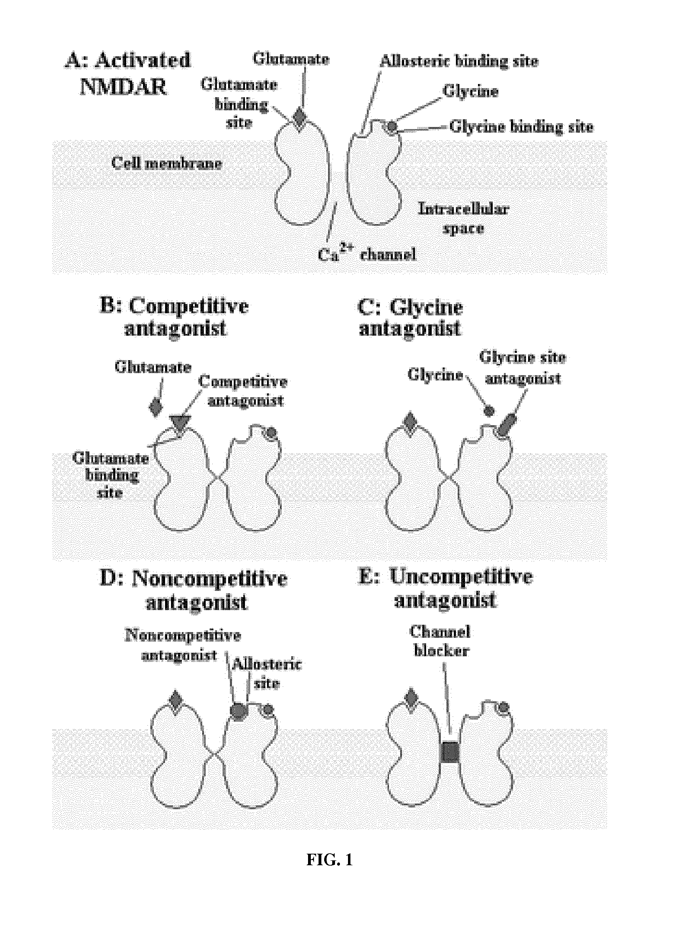

- **"NMDA receptor antagonist"** or **"antagonizing NMDA receptor"** refers to a compound that reduces the flow of cations (Na⁺, K⁺, Ca²⁺) through the NMDA receptor. The NMDA receptor antagonists comprise four categories of compounds: competitive antagonists, which bind to and block the binding site of the neurotransmitter glutamate; glycine antagonists, which bind to and block the glycine site; noncompetitive antagonists, which inhibit NMDA receptors by binding to allosteric sites; and uncompetitive antagonists or channel blockers, which block the ion channel by binding to a site within it.
- **"NMDA receptor agonist"** refers to a compound that increases the flow of cations through the NMDA receptor. NMDA receptor requires the binding of glycine and glutamate to open and is then considered as "activated" **(****Figure 1A****).**
- "**Noncompetitive antagonists"** refer to compounds which require the binding of glycine and glutamate then said compound can bind to an allosteric site of the channel and block the flow of cations **(****Figure 1D****).**
- **"Uncompetitive antagonists"** refer to compounds which require the binding of an agonist of the NMDA receptor (e.g. glycine, glutamate) and the channel opening to access their blocking site **(Figure IE).** The uncompetitive channel blocker then becomes trapped within the NMDA receptor.
- **"Analog"** refers broadly to the modification or substitution of one or more chemical moieties on a parent compound and may include functional derivatives, positional isomers, tautomers, zwitterions, enantiomers, diastereomers, racemates, isosteres or stereochemical mixtures thereof.
- **"Functional derivative"** refers to a compound which possesses similar IC50 values and kinetics properties as ketamine and memantine to NMDA receptor. The functional derivative of the invention possesses the capacity to restore attention, suppress and/or decrease impulsivity and/or aggressiveness.
- **"Binding"** can mean, but is in no way limited to, the physical or chemical interaction, direct or indirect, between two molecules (e.g., compounds, amino acids, nucleotides, polypeptides, or nucleic acids). Binding includes covalent, hydrogen bond, ionic, non-ionic, van der Waals, hydrophobic interactions, and the like.
- **"Sub-anesthetic dose"** refers to dosage of an NMDA antagonist or an analog or a functional derivative thereof to be administered to a subject without causing any loss of consciousness; any attendant risks should be very small. This dosage enhances an analgesic effect.
- **"Pharmaceutically acceptable excipient"** refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, and general safety and purity standards as required by FDA Office of Biologics standards.
- **"Treatment"** refers to therapeutic treatment, prophylactic or preventative measures and deferment of the disease onset; wherein the object is to delay, prevent or slow down (lessen) the targeted pathologic condition or disorder. Those in need of treatment include those already with mental disorders with occurrence of aggressive and/or impulsive behavior, as well as those prone to have mental disorders with occurrence of aggressive and/or impulsive behavior, or those in whom mental disorders with occurrence of aggressive and/or impulsive behavior is to be prevented or delayed. A subject or mammal is successfully "treated" for mental disorders with occurrence of aggressive and/or impulsive behavior if, after receiving a sub-anaesthetic dose of a composition according to the invention, the patient shows observable and/or measurable attention improvements, decrease in impulsive and/or aggressive behavior and/or improvement in quality of life issues. The above parameters for assessing successful treatment and improvement in the disease are readily measurable by routine procedures familiar to a physician.
- **"Subject"** refers to a mammal, preferably a human. In one embodiment, the subject is female. In another embodiment, the subject is male.
- **"About":** preceding a figure means plus or less 10% of the value of said figure.

### DETAILED DESCRIPTION

This invention relates to a composition for use in the treatment of a mental disorder with occurrence of aggressive and/or impulsive behavior comprising a sub-anesthetic dose of a compound antagonizing NMDA receptors.

The term "compound" as used throughout the specification includes but is limited to: active metabolite, enantiomer, isomer, functional derivative, analog or salt of the NMDA antagonist of the invention.

The NMDA receptor is an ionotropic receptor that allows for the transfer of electrical signals between neurons in the brain and in the spinal column. For electrical signals to pass, the NMDA receptor must be open. To remain open, glutamate and glycine must bind to the NMDA receptor. An NMDA receptor that has glycine and glutamate bound to it and has an open ion channel is called "activated". Chemicals that deactivate the NMDA receptor are called antagonists.

Each receptor subunit has modular design and each structural module also represents a functional unit: the extracellular domain contains two globular structures: a modulatory domain and a ligand-binding domain. NR1 subunits bind the co-agonist glycine and NR2 subunits bind the neurotransmitter glutamate. The agonist-binding module links to a membrane domain, which consists of three trans-membrane segments and a re-entrant loop reminiscent of the selectivity filter of potassium channels. The membrane domain contributes residues to the channel pore and is responsible for the receptor's high-unitary conductance, high-calcium permeability, and voltage-dependent magnesium block. Each subunit has an extensive cytoplasmic domain, which contain residues that can be directly modified by a series of protein kinases and protein phosphatases, as well as residues that interact with a large number of structural, adaptor, and scaffolding proteins.

Activation of NMDA receptors results in the opening of an ion channel that is nonselective to cations. A unique property of the NMDA receptor is its voltage-dependent activation, a result of ion channel block by extracellular Mg²⁺ ions. This allows voltage-dependent flow of Na⁺ and small amounts of Ca²⁺ ions into the cell and K⁺ out of the cell. Activation of NMDA receptors requires binding of glutamate or aspartate (aspartate does not stimulate the receptors as strongly). In addition, NMDA receptors also require the binding of the co-agonist glycine for the efficient opening of the ion channel, which is a part of this receptor.

In one embodiment, the compound of the invention has similar selectivity, IC₅₀ values or kinetics properties for the NMDA receptor subtypes than ketamine or memantine.

In another embodiment, the compound of the invention has an IC₅₀ value inferior to 2 µM for the subunit GluN2C of the NMDA receptor and inferior to 3 µM for the subunit GluN2D of the NMDA receptor. IC₅₀ values or kinetics properties determination are well known by the skilled artisan. For example, the IC₅₀ of a drug can be determined by constructing a dose-response curve and examining the effect of different concentrations of antagonist on reversing agonist activity.

In another embodiment, the compound of the invention requires the binding of an NMDA agonist and channel opening to access to its binding, allosteric and/or blocking site.

In one embodiment of the invention, the compound inhibits the receptor by binding both in the internal portion of the pore and can remain trapped inside the channel following its closure.

In one embodiment of the invention, the compound is noncompetitive antagonist of the NMDA receptor. Examples of noncompetitive antagonists include but are not limited to: ketamine; amantadine; tiletamine; phencyclidine (PCP); PCP hydrochloride functional derivatives; dizocilpine MK-801; Argiotoxin 636, dextrorphan, HU-211, Rhynchophylline and/or analogs and/or functional derivatives thereof.

In another embodiment, the compound is an uncompetitive channel blocker of the NMDA receptor. Examples of uncompetitive antagonists of the NMDA receptor include but are not limited to: memantine; aptiganel or Cerestat or CNS-1102, ifenprodil, lanicemine, Remacemide and/or analogs and/or functional derivatives thereof.

There are many different conditions that are recognized as mental disorders with occurrence of aggressive and/or impulsive behavior or psychiatric disorders with occurrence of aggressive and/or impulsive behavior.
- Personality disorders: People with personality disorders have extreme and inflexible personality traits that are distressing to the person and/or cause problems in work, school, or social relationships. In addition, the person's patterns of thinking and behavior significantly differ from the expectations of society and are so rigid that they interfere with the person's normal functioning. Examples of personality disorders include but are not limited to: obsessive-compulsive personality disorder, attention deficit hyperactivity disorder (ADHD) and attention deficit disorder (ADD), Tourette syndrome, Tic disorder, antisocial personality disorder, and paranoid personality disorder.
- Anxiety disorders: People with anxiety disorders respond to certain objects or situations with fear and dread, as well as with physical signs of anxiety or nervousness, such as a rapid heartbeat and sweating. An anxiety disorder is diagnosed if the person's response is not appropriate for the situation, if the person cannot control the response, or if the anxiety interferes with normal functioning. Examples of anxiety disorders include but are not limited to: generalized anxiety disorder, post-traumatic stress disorder (PTSD), obsessive-compulsive disorder (OCD), panic disorder, social anxiety disorder, and specific phobias.
- Mood disorders: These disorders, also called affective disorders, involve persistent feelings of sadness or periods of feeling overly happy, or fluctuations from extreme happiness to extreme sadness. Examples of mood disorders include but are not limited to: Intermittent explosive disorder (IED), depression, mania, and bipolar disorder.
- Impulse control and addiction disorders: People with impulse control disorders are unable to resist urges, or impulses, to perform acts that could be harmful to themselves or others. Often, people with these disorders become so involved with the objects of their addiction that they begin to ignore responsibilities and relationships. Examples of impulse control and addiction disorders include but are not limited to: IED, Pyromania (starting fires), kleptomania (stealing), and compulsive gambling are examples of impulse control disorders. Alcohol and drugs are common objects of addictions.

The present invention also relates to a pharmaceutical composition for treating a mental disorder with occurrence of aggressive and/or impulsive behavior in a subject in need thereof, wherein said pharmaceutical composition comprises a sub-anesthetic dose of an NMDA antagonist and at least one pharmaceutically acceptable excipient.

Another object of the invention is a medicament for treating a mental disorder with occurrence of aggressive and/or impulsive behavior comprising a sub-anesthetic dose of an NMDA antagonist.

In one embodiment, the compound of this invention may be provided in the form of a pharmaceutically acceptable salt. Examples of such a salt include, but are not limited to, those formed with organic acids (e.g. acetic, lactic, citric, malic, formaric, tartaric, stearic, ascorbic, succinic, benzoic, methanesulfonic, toluenesulfonic, or pamoic acid), inorganic acids (e.g., hydrochloridic, nitric, diphosphoric, sulphuric, or phosphoric acid), and polymeric acids (e.g., tannic acid, carboxymethyl cellulose, polylactic, polyglycolic, or co-polymers of polylactic-glycolic acids).

In another embodiment, the composition, pharmaceutical composition or medicament of the invention further comprises some excipients, such as, for example, surfactants (e.g. hydroxypropylcellulose); suitable carriers, such as, for example, solvents and dispersion media containing, for example, water, monosodium or disodium phosphate, sodium, potassium, calcium or magnesium chloride, ethanol, polyol (e.g. glycerol, propylene glycol, and liquid polyethylene glycol, and the like), suitable mixtures thereof, and vegetable oils, such as, for example, peanut oil and sesame oil; isotonic agents, such as, for example, sugars or sodium chloride; coating agents, such as, for example, lecithin; agents delaying absorption, such as, for example, aluminum monostearate and gelatin; preservatives, such as, for example, benzalkonium chloride, benzethonium chloride, chlorobutanol, thimerosal and the like; buffers, such as, for example, boric acid, sodium and potassium bicarbonate, sodium and potassium borates, sodium and potassium carbonate, sodium acetate, sodium biphosphate and the like; tonicity agents, such as, for example, dextran 40, dextran 70, dextrose, glycerin, potassium chloride, propylene glycol, sodium chloride; antioxidants and stabilizers, such as, for example, sodium bisulfite, sodium metabisulfite, sodium thiosulfite, thiourea and the like; nonionic wetting or clarifying agents, such as, for example, polysorbate 80, polysorbate 20, poloxamer 282 and tyloxapol; viscosity modifying agents, such as, for example dextran 40, dextran 70, gelatin, glycerin, hydroxyethylcellulose, hydroxmethylpropylcellulose, lanolin, methylcellulose, petrolatum, polyethylene glycol, polyvinyl alcohol, polyvinylpyrrolidone, carboxymethylcellulose; and the like.

In one embodiment of the invention, the composition, the pharmaceutical composition or the medicament comprises a sub-anesthetic dose of an NMDA antagonist.

In one embodiment, the administration to a subject in need thereof of the composition of the invention results in the alleviation of symptoms of mental disorders with occurrence of aggressive and/or impulsive behavior.

According to the invention, the sub-anesthetic dose of an NMDA antagonist is calculated in order to reach a desired behavior such as improvement in attention level, decrease in impulsivity and/or aggressiveness. Methods for assessing the efficacy of the treatment are readily measurable by routing procedures familiar to a physician.

The composition, pharmaceutical composition or medicament of the invention may be administered by several routes of administration. Examples of adapted routes of administration include, but are not limited to: intramuscular (i.m.), subcutaneous, intravenous, intraocular, transdermal, topical, parenteral, intranasal and oral administration. Parenteral administration may be by intravenous (IV) injection, subcutaneous (s.c.) injection, intramuscular (i.m) injection, intra-arterial injection, intrathecal (i.t.) injection, intra-peritoneal (i.p.) injection, or direct injection or other administration to the subject.

In one embodiment of the invention, the sub-anesthetic dose of the NMDA antagonist is inferior or equal to 15 mg/kg body weight/day, 14, 13, 12, 11, 10, 9, 8, 7, 6, 5, 4, 3, 2, 1 mg/kg body weight/day.

In one embodiment of the invention, the sub-anesthetic dose of an NMDA antagonist is inferior to 0.5 mg/kg body weight/day, preferably inferior to 0.25 mg/kg.

It will be understood that the sub-anaesthetic dose of the compounds and compositions of the present invention will be decided by the attending physician within the scope of sound medical judgment. The specific sub-anaesthetic dose for any particular subject will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific peptide employed; and like factors well known in the medical arts. For example, it is well known within the skill of the art to start doses of the compound at levels lower than those required to achieve the desired therapeutic effect and to gradually increase the dosage until the desired effect is achieved.

According to an embodiment, the composition, pharmaceutical composition or medicament of the invention is in a form adapted for injection, preferably selected from the group comprising solutions, such as, for example, isotonic solution, saline solution, sterile aqueous solutions, dispersions, emulsions, suspensions, solid forms suitable for using to prepare solutions or suspensions upon the addition of a liquid prior to use, such as, for example, powder, freeze-dried compositions, liposomal forms and the like. Sterile injectable solutions are prepared by incorporating the composition of the invention in the required amount in the appropriate solvent with various of the other ingredients enumerated above, as required, followed by filtered sterilization. Generally, dispersions are prepared by incorporating the various sterilized composition into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

According to an embodiment, the composition, pharmaceutical composition or medicament of the invention is in a form adapted to oral administration. According to a first embodiment, the form adapted to oral administration is a solid form selected from the group comprising tablets, pills, capsules, soft gelatin capsules, sugar-coated pills, orodispersing tablets, effervescent tablets or other solids. According to a second embodiment, the form adapted to oral administration is a liquid form, such as, for example, a drinkable solution, a buccal spray, liposomal forms and the like. The oral drug components are combined with any oral, non-toxic, pharmaceutically acceptable inert carrier such as ethanol, glycerol, water, and the like. Examples of suitable liquid dosage forms include solutions or suspensions in water, pharmaceutically acceptable fats and oils, alcohols or other organic solvents, including esters, emulsions, syrups or elixirs, suspensions, solutions and/or suspensions reconstituted from non-effervescent granules and effervescent preparations reconstituted from effervescent granules. Such liquid dosage forms may contain, for example, suitable solvents, preservatives, emulsifying agents, suspending agents, diluents, sweeteners, thickeners, and melting agents.

According to an embodiment, the composition, pharmaceutical composition or medicament of the invention is in a form adapted for local delivery via the nasal and respiratory routes. Examples of formulations suitable for nasal administration include but are not limited to, nasal solutions, sprays, aerosols and inhalants.

According to an embodiment, the composition, pharmaceutical composition or medicament of the invention is in the form of, or comprises, liposomes and/or nanoparticles.

In one embodiment, the composition of the invention is in a form adapted for a topical administration.

In one embodiment, the composition of the invention is formulated as a patch for topical or transdermal administration.

Examples of formulations adapted to topical administration include, but are not limited to: patches, sticks, lipsticks, waxes, creams, lotions, ointments, balms, gels, glosses, masks, leave-on washes or cleansers and/or the like.

In one embodiment, the composition of the invention can be mixed to form white, smooth, homogeneous, opaque cream or lotion with, for example, benzyl alcohol 1 % or 2% (wt/wt) as a preservative, emulsifying wax, glycerin, isopropyl palmitate, lactic acid, purified water and sorbitol solution. In addition, the compositions can contain polyethylene glycol 400. They can be mixed to form ointments with, for example, benzyl alcohol 2% (wt/wt) as preservative, white petrolatum, emulsifying wax, and tenox II (butylated hydroxyanisole, propyl gallate, citric acid, propylene glycol). Woven pads or rolls of bandaging material, e.g. gauze, can be impregnated with the compositions in solution, lotion, cream, ointment or other such form can also be used for topical application.

In another embodiment of the invention, the composition of the invention is in a form adapted for transdermal administration.

In one embodiment, the transdermal composition is an ointment, paste, cream; film, balm, patch, such as, for example, transdermal patch, gel, liposomal forms or the like.

In one embodiment of the invention, the ointment is an oleaginous ointment; an emulsified ointment such as, for example, oil-in-water or a water-in-oil ointment; or a water-soluble ointment, preferably is an oleaginous ointment.

In one embodiment of the invention, the oleaginous ointment uses bases such as, for example, plant and animal oils; plant and animal fats; waxes; Vaseline, such as, for example, white Vaseline or Vaseline oil; and paraffin such as, for example, liquid paraffin or paraffin oil.

In one embodiment of the invention, the transdermal composition further comprises one or more excipients. Suitable pharmaceutically acceptable excipients are well known from the skilled person. Examples of suitable excipients include, but are not limited to, carriers, emulsifying agents, stiffening agents, rheology modifiers or thickeners, surfactants, emollients, preservatives, humectants, buffering agents, solvents, moisturizing agents and stabilizers.

Those of skill in the art are well aware of general technologies for administration of transdermal or topical composition. Transdermal drug delivery offers controlled release of a drug to the patient and transdermal patches are user-friendly, convenient, painless, and offer multi-day dosing which usually results in improved patient compliance.

Such composition can be administered to the skin of the face, scalp, temporal region, arms, stomach, thighs, back, neck and the like. Suitable skin of the face includes skin of the chin, the upper lip, the lower lip, the forehead, the nose, the cheek, the skin around the eyes, the upper eyelid, the lower eyelid or combinations thereof. Suitable skin of the scalp includes the front of the scalp, the scalp over the temporal region, the lateral part of the scalp, or combinations thereof. Suitable skin of the temporal region includes the temple and the scalp over the temporal region and combinations thereof. The composition may be formulated into a bioadhesive patch or a bioadhesive strip with an occlusive covering. Alternatively, the transdermal composition for administration to the skin can be applied as a topical ointment, a topical gel, a lotion, a cream, a solution, a spray, a paint, a varnish, a film, a foil, to be applied to the skin in a layer with or without an occlusive dressing.

In another embodiment, the composition of the invention can also be applied topically using a transdermal system, such as one of an acrylic-based polymer adhesive with a resinous crosslinking agent impregnated with the composition and laminated to an impermeable backing.

The present invention also relates to a patch comprising the composition of the invention, more particularly as a sustained-release patch. Patches can include any conventional form such as, for example, adhesive matrix, polymeric matrix, reservoir patch, matrix or monolithic-type laminated structure, and are generally comprised of one or more backing layers, adhesives, penetration enhancers, an optional rate controlling membrane and a release liner which is removed to expose the adhesives prior to application. Polymeric matrix patches also comprise a polymeric-matrix forming material. Suitable transdermal patches are described in more detail in, for example, U. S. Patent Nos. 5,262,165; 5,948,433; 6,010,715 and 6,071,531; the disclosure of each of which are incorporated herein in their entirety.

In one embodiment, the composition of the invention is to be administered in a sustained-release form.

Sustained-release preparations may be prepared. Suitable examples of sustained-release preparations include semipermeable matrices of solid hydrophobic polymers containing memantine, which matrices are in the form of shaped articles, e.g., patches, films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, nondegradable ethylene-vinyl acetate, degradable lactic acid-glycolic acid copolymers such as the Lupron Depot™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37° C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

The formulations of the invention may be designed to be short-acting, fast-releasing, long-acting, or sustained-releasing as described herein. Thus, the pharmaceutical formulations may also be formulated for controlled release or for slow release.

In one embodiment of the invention, the composition comprises a delivery system that controls the release of the composition. Examples of suitable carriers for sustained or delayed release include, but are not limited to, gelatin; gum Arabic; xanthane polymers; thermoplastic resins such as, for example polyvinyl halides, polyvinyl esters, polyvinylidene halides and halogenated polyolefins; elastomers such as, for example, brasiliensis, polydienes, and halogenated natural and synthetic rubbers; and flexible thermoset resins such as polyurethanes, epoxy resins; biodegradable polymers and the like.

In one embodiment of the invention, the composition, pharmaceutical composition or medicament of the invention may be used in conjunction with delivery systems that facilitate delivery of the agents to the central nervous system. For example, various blood brain barrier (BBB) permeability enhancers may be used to transiently and reversibly increase the permeability of the blood brain barrier to a treatment agent. Such BBB permeability enhancers include but are not limited to leukotrienes, bradykinin agonists, histamine, tight junction disruptors (e.g., zonulin, zot), hyperosmotic solutions (e.g., mannitol), cytoskeletal contracting agents, and short chain alkylglycerols (e.g., 1-O-pentylglycerol). Oral, sublingual, parenteral, implantation, nasal and inhalational routes can provide delivery of the active agent to the central nervous system. In some embodiments, the compounds of the present invention may be administered to the central nervous system with minimal effects on the peripheral nervous system.

The blood-brain barrier (BBB) is a physical barrier and system of cellular transport mechanisms between the blood vessels in the central nervous system (CNS) and most areas of the CNS itself. The BBB maintains homeostasis by restricting the entry of potentially harmful chemicals from the blood, and by allowing the entry of essential nutrients. However, the BBB can pose a formidable barrier to delivery of pharmacological agents to the CNS for treatment of disorders or maintaining or enhancing normal and desirable brain functions, such as cognition, learning, and memory.

The present invention can also relate to a prodrug of the NMDA antagonist or an encapsulation of said antagonist.

In one embodiment, the composition, pharmaceutical composition or medicament of the invention is a prodrug of the selective NMDA antagonist.

In another embodiment, the composition, pharmaceutical composition or medicament of the invention is a prodrug of the ketamine and/or analog and/or functional derivative thereof.

Prodrugs as described herein are capable of passage across the blood-brain barrier and may undergo hydrolysis by CNS esterases to provide the active compound.

Prodrugs provided herein may also exhibit improved bioavailability, improved aqueous solubility, improved passive intestinal absorption, improved transporter-mediated intestinal absorption, protection against accelerated metabolism, tissue-selective delivery, less (or fewer) side effects, lessened or no deleterious drug interaction with other medications, and/or passive enrichment in the target tissue.

The term "prodrug" as used herein refers to a compound that is converted under physiological conditions, by solvolysis or metabolically to a specified compound that is pharmaceutically/pharmacologically active. The "prodrug" can be a compound of the present invention that has been chemically derivatized such that it retains some, all or none of the bioactivity of its parent drug compound and is metabolized in a subject to yield the parent drug compound. The prodrug of the present invention may also be a "partial prodrug" in that the compound has been chemically derivatized such that it retains some, all or none of the bioactivity of its parent drug compound and is metabolized in a subject to yield a biologically active derivative of the compound.

Prodrugs can be formed by attachment of biocompatible polymers, such as those previously described including polyethylene glycol (PEG), to compounds of the present invention using linkages degradable under physiological conditions. See also Schacht, et al. (1997) Poly(ethylene glycol) Chemistry and Biological Applications, American Chemical Society, San Francisco, CA 297-315. Attachment of PEG to proteins can be employed to reduce immunogenicity and/or extend the half-life of the compounds provided herein. Any conventional PEGylation method can be employed, provided that the PEGylated agent retains at least some pharmaceutical activity.

In one embodiment, the selective antagonist of the invention is PEGylated.

In one embodiment, the present invention further provides prodrugs comprising the compounds described herein. The prodrugs can be formed utilizing a hydrolyzable coupling to the compounds described herein. Ettmayer, et al. (2004) J. Med. Chem. 47(10): 2394-2404; Testa and Mayer (2003) Hydrolysis in Drug and Prodrug Metabolism: Chemistry, Biochemistry and Enzymology Wiley-Verlag Helvetica Chimica Acta, Zuerich (Chapters 1-1 ): 1-780.

In one embodiment, the composition, the pharmaceutical composition or the medicament of the invention is sterile. Advantageously, it comprises a preservative in order to prevent the growth of microorganisms. The prevention of the action of microorganisms may be brought about by various antibacterial and antifungal agents, such as, for example, parabens, chlorobutanol, phenol, sorbic acid, thimerosal, and the like.

In one embodiment, the sub-anesthetic dose of the compound of the invention is administered once a month, twice a month, once a week, twice a week, at least once a day, twice, or three times a day.

In another embodiment, the sub-anesthetic dose of the compound of the invention is administered once a day on consecutive days for at least a week, at least a month, at least a year, or on as needed basis for the rest of the patient's life.

In another embodiment, the sub-anesthetic dose of the compound of the invention is administered once a week on consecutive weeks for at least two weeks, one month, at least a year, or more as needed basis for the rest of the patient's life.

In another embodiment, the sub-anesthetic dose of the compound of the invention is administered once a month on consecutive months for at least two months, a year, or more as needed basis for the rest of the patient's life.

According to one embodiment of the invention, the administration dose of the pharmaceutical composition is determined by the skilled artisan and personally adapted to each subject.

In one embodiment of the invention, sub-anesthetic dose of the compound of the invention is to be administered for a chronic treatment.

In another embodiment of the invention, sub-anesthetic dose of the compound of the invention is to be administered for an acute treatment.

In one embodiment, the composition, the pharmaceutical composition or the medicament of the invention is to be administered alone, i.e. is not administered in combination with another therapeutic agent for treating mental disorder with occurrence of aggressive and/or impulsive behavior.

In one embodiment, the composition, the pharmaceutical composition or the medicament of the present invention is to be administered with other active agents. In one embodiment, the composition, the pharmaceutical composition or the medicament and the other active agent may be administered separately or in conjunction with the compound of the invention.

In one embodiment of the invention, the subject is a young child. As used herein, the term "young child" refers to a child from 0 to 3 years old.

In another embodiment of the invention, the subject is a child. In one embodiment, the term "child" may refer to subjects aged from 0 to 12, preferably from 3 to 12. More generally, the term child refers to a subject which is not yet an adolescent.

In another embodiment of the invention, the subject is an adolescent. In one embodiment, the term "adolescent" may refer to subjects aged from about 12 to 17, but the skilled artisan will appreciate that the length of adolescence may vary from one individual to another.

In another embodiment, the subject is an adult. In one embodiment, the term "adult" may refer to subjects of more than 17 years old. More generally, the term adult refers to a subject which is no more an adolescent.

In one embodiment of the invention, the subject to be treated is diagnosed with a mental disorder with occurrence of aggressive and/or impulsive behavior.

In another embodiment, the subject diagnosed with a mental disorder with occurrence of aggressive and/or impulsive behavior is already treated with other active agents as described herein to alleviate symptoms of a mental disorder with occurrence of aggressive and/or impulsive behavior.

In another embodiment of the invention, the subject to be treated is at risk of developing a mental disorder with occurrence of aggressive and/or impulsive behavior.

Increased risks factors increasing the development of mental disorders with occurrence of aggressive and/or impulsive behavior, include but are not limited to: parental treatment such as parental rejection, lack of parental warmth, high hostility, harsh discipline, high maternal negative affect, anxious childrearing, modelling of dysfunctional and drug-abusing behavior, and child abuse (emotional, physical and sexual), migration and discrimination, childhood trauma, bereavement or separation in families, drug abuse, family history (e.g. of anxiety), temperament and attitudes.

In one embodiment of the invention, the subject has a genetic or a familial predisposition to a mental disorder with occurrence of aggressive and/or impulsive behavior.

Examples of genetic predisposition include but are not limited to: linkage to chromosome bands 16p13, on chromosome 2p23.2, mutations involving genes dopamine receptor D3 (DRD)3, DRD4, dopamine active transporter (SLC6A3) (DAT1), dopamine beta-hydroxylase (DBH), SLIT and NTRK-like family, member 1 (SLITRK1).

The present invention also relates to a method for treating mental disorders with occurrence of aggressive and/or impulsive behavior in a subject in need thereof, wherein said method comprises administering to the subject in need thereof of a sub-anesthetic dose of an NMDA antagonist.

In one embodiment of the invention, said sub-anesthetic dose is calculated in order to reach a desired behavior such as good attention level, less impulsivity and/or less aggressiveness.

In one embodiment of the invention, the method for treating mental disorders with occurrence of aggressive and/or impulsive behavior in a subject in need thereof comprises a chronic treatment.

In another embodiment of the invention, the method for treating mental disorders with occurrence of aggressive and/or impulsive behavior in a subject in need thereof comprises an acute treatment.

In one embodiment of the invention, the method of treating comprises administering to the subject the composition, the pharmaceutical composition or the medicament of the invention.

In one embodiment of the invention, the method is for treating behavioral and/or cognitive symptoms of mental disorders with occurrence of aggressive and/or impulsive behavior.

Another object of the present invention is a method for inhibiting visually-guided saccades in a subject in need thereof comprising the administration of a sub-anesthetic dose of the compound of the invention.

Another object of the present invention is a method for increasing the latency of anticipatory saccades in a subject in need thereof comprising the administration of sub-anesthetic dose of the compound of the invention.

Another object of the present invention is a method for reducing the occurrence of saccades in a subject in need thereof comprising the administration of sub-anesthetic dose of the compound of the invention.

Another object of the present invention is a method for reducing anticipatory or premature saccades occurrence in a subject in need thereof comprising the administration of sub-anesthetic dose of the compound of the invention.

### BRIEF DESCRIPTION OF THE DRAWINGS

Figure 1 is a diagram showing a simplified model of NMDA receptor channel activation and various types of antagonists and/or blockers.

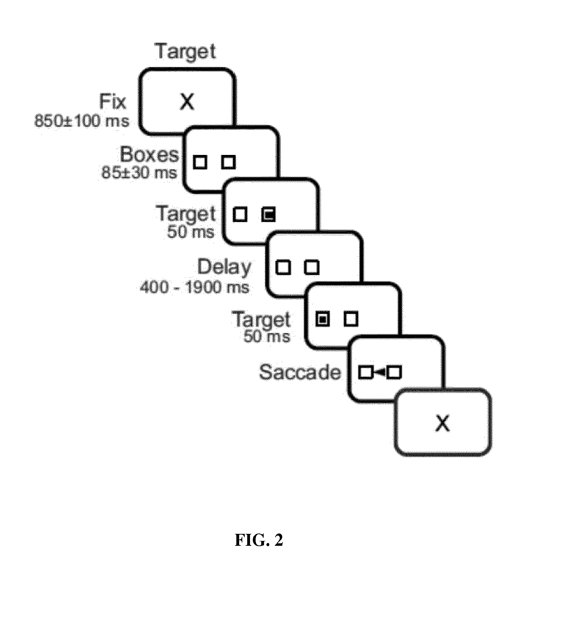

Figure 2 is a diagram showing the sequence of events. The trial started with the appearance of a fixation cross for a randomized duration followed by the appearance of two empty 'boxes', one at the center of the screen and at a 9-deg eccentric position. After the appearance of the two boxes, a target was flashed in the central one for 50 milliseconds. Extinction of the central target marked the beginning of the delay period that could last either 400, 900, 1400 or 1900 ms. At the end of the delay period, a target appeared for 50 ms in the eccentric box and the subject had to make a saccade to the eccentric box within a 400 ms grace period.
Figure 3 represents the influence of ketamine (solid curve) vs control (dashed curve) i.m. injections on saccadic latency. Group data. A: All saccades. B: Visually guided saccades only (latency >100 ms). C: Premature saccades (latency < 100 ms). X-axis, delay duration; Y, axis, mean saccadic latency.
Figure 4 represents the cumulated latency distribution for the 4 delay durations tested. Group data. The vertical dashed line represents 100 ms latency. Saccades with a latency shorter than 100 ms were considered as premature.
Figure 5 represents the number of premature (dashed curve) and visually-guided (solid curve) saccades as a function of delay duration and treatment in the two monkeys generating significant amounts of premature movements.
Figure 6 represents the variance of latency distributions as a function of delay duration and treatment (grey curve).
Figure 7 represents the time course of the influence of ketamine. A: saccadic latency as a function of trial number before (black) and after (grey) ketamine injection. Monkey Y.
Single injection. Note the sharp decrease of premature saccades after ketamine injection (vertical grey arrow). B: Saccadic latency as a function of trial number, last 200 trials of the control block and first 200 trials after injection of ketamine. Monkeys Y and L. Data from the 13 injections sites are represented. C: the cumulated sum of latencies for both control and ketamine.

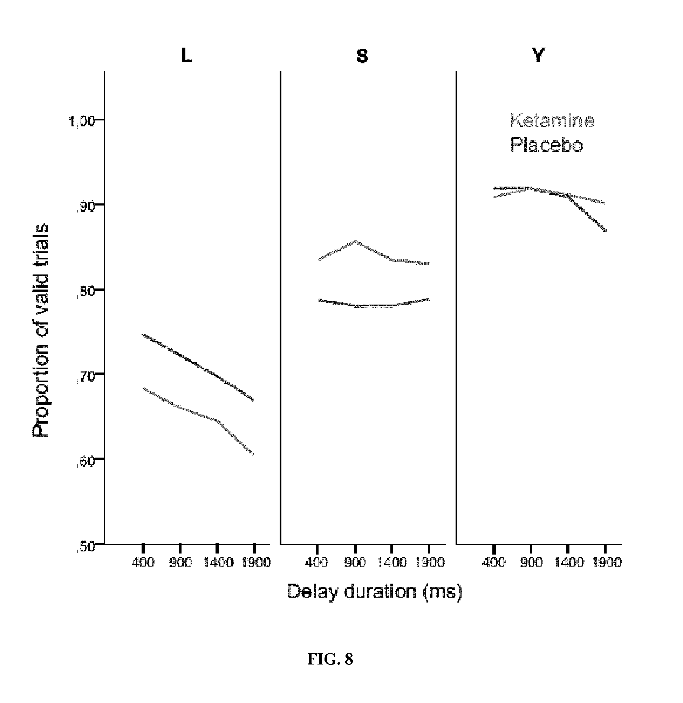

Figure 8 represents the proportion of valid trials as a function of delay duration and drug treatment (grey curves) for the three monkeys of the present study.

### EXAMPLES

The present invention is further illustrated by the following examples.

### Example 1: Influence of ketamine on saccade latency

The experimental paradigm was designed to create a conflict between the urge to make a saccade to the peripheral box and the necessity to maintain fixation until the eccentric target appeared **(****Figure 2****).** This required a strong inhibition of premature saccades before the appearance of the eccentric target.

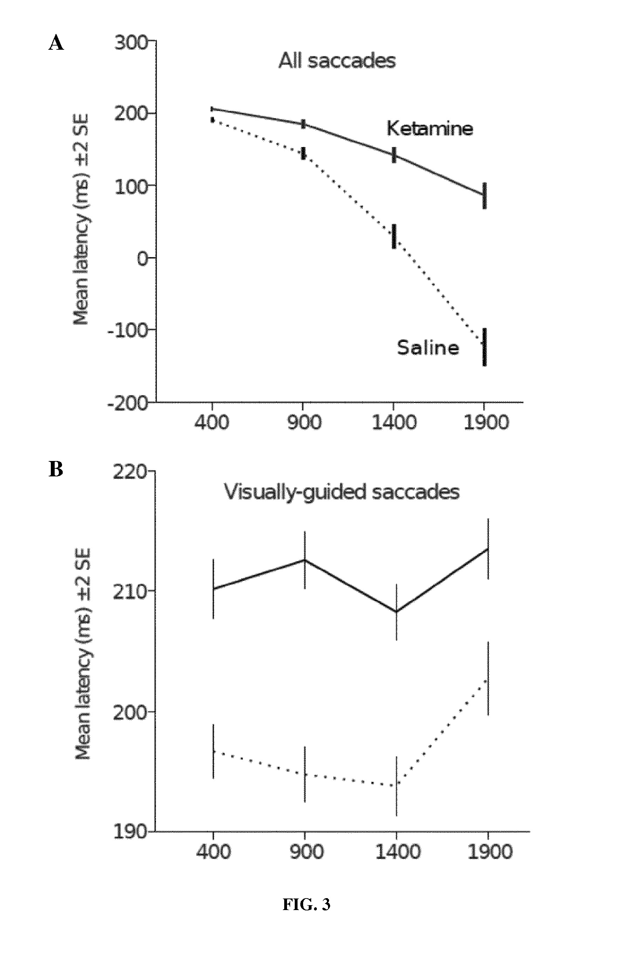

A total of 14104 saccades were analyzed in the three monkeys of the present study (L, S, Y). We found that a 0.25 mg/kg dose of ketamine altered saccadic latency. **Figure 3A** shows the influence of ketamine or saline injection (placebo, vehicle) on saccade latency during the two blocks of trials following injection (group data of the three subjects). Time zero on the ordinate shows the time of target appearance at the eccentric position. Positive values represent saccades occurring after target onset. We classified saccades as visually-guided for latencies longer than 100 ms. Latencies shorter than 100 ms represent premature (anticipatory) saccades. It can be observed that in the control condition (dashed curve on **Figure 3A**), average saccadic latencies tended on average to decrease with increasing delay duration, more premature saccades occurring during long delays. However, after ketamine injection, saccadic latencies only modestly decreased with increasing delay duration (solid curve). In group data, an ANOVA with delay duration and injected product as fixed factors did not reveal a significant difference between latencies of saccades in the control (n=6529 saccades) and ketamine (n=7575 saccades) conditions (F_{1; 2.001}=11.275; P=0.078). However, a significant interaction between injected product (placebo or ketamine) and delay duration was found (F_{3; 6.012} = 8.195; P=0.015). Ketamine prolonged saccadic latencies differently with increasing delay duration. Saccades in short delay trials (400 or 900 ms delays) were less affected than saccades when the delay was longer (1400 or 1900 ms). This influence of ketamine on movement latency was reproducible between monkeys. Indeed, there was no significant interaction between injected product and the random factor (monkey identity) on saccadic latency (F_{2;6.001}=3.647; p=0.092). **Table 1** summarizes observed effects.

### Dependent Variable: Saccadic latency (ms)

**Table 1. Summary of observed effects.**

| Source | | Type III Sum of Squares | df | Mean Square | F | P |
|---|---|---|---|---|---|---|
| Intercept | Hypo | 148515263,918 | 1 | 148515263,918 | 3,796 | ,191 |
| | Error | 78254200,322 | 2,000 | 39126186,084^{a} | | |
| duration | Hypo | 106769980,334 | 3 | 35589993,445 | 3,331 | ,098 |
| | Error | 64106718,332 | 6,001 | 10683503,890^{b} | | |
| product | Hypo | 20350449,302 | 1 | 20350449,302 | 11,275 | ,078 |
| | Error | 3611533,788 | 2,001 | 1804852,486^{c} | | |
| monkey | Hypo | 78821874,412 | 2 | 39410937,206 | 3,264 | ,100 |
| | Error | 83930755,420 | 6,952 | 12073515,510^{d} | | |
| duration * product | Hypo | 12183518,654 | 3 | 4061172,885 | 8,195 | ,015 |
| | Error | 2978970,135 | 6,012 | 495544,556^{e} | | |
| duration * monkey | Hypo | 64583469,278 | 6 | 10763911,546 | 21,579 | ,001 |
| | Error | 2992909,356 | 6 | 498818,226^{f} | | |
| product * monkey | Hypo | 3635097,299 | 2 | 1817548,650 | 3,647 | ,092 |
| | Error | 2991259,218 | 6,001 | 498430,887^{g} | | |
| duration * product * | Hypo | 2992909,356 | 6 | 498818,226 | 7,898 | ,000 |
| monkey | Error | 889206326,916 | 14080 | 63153,858^{h} | | |

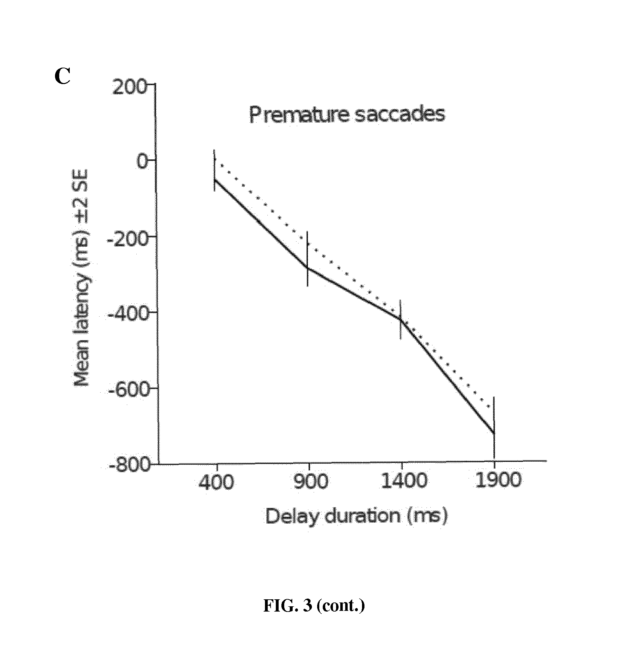

As shown above, negative average saccadic latencies in controls (blues curve on **Figure 3A****)** suggest that the effect of ketamine might be more pronounced on premature saccades rather than visually-guided saccades. Therefore, we analyzed separately the influence of ketamine injection on premature saccades (with latencies shorter than 100 ms) and visually-guided saccades (latencies longer than 100 ms). **Figure 3B** shows the influence of ketamine injection on visually-guided saccades only. The average difference in the latency of visually-guided saccade between conditions was rather small (approx. 10 ms). An ANOVA with injected product and delay duration as fixed factors did not reveal a significant main effect of ketamine (F_{1;2.002}=5.3; p=0.148) or a significant interaction with delay duration (F_{3,6.069}=0.154; p=0.923). However, there was a significant interaction between injected product and the random factor monkey identity (F_{2; 6.042}=11.081; p=0.01). Although there was an influence of ketamine on visual saccadic latencies in each monkey, this effect was so variable between subjects that it did not reach significance at the group level. **Figure 3C** shows the influence of delay duration and ketamine on premature saccades (latencies less than 100 ms). Delay duration had a strong and significant effect on movement latency (F_{3; 21.703}=47.319; p=0.000). However, here also, ketamine did not significantly alter saccade latency (F_{1; 5.464}=1.219; p=0.316). There was no significant interaction between fixed factors (F_{3; 25.033}=0.636; p=0.599) and the random factor (monkey identity) did not play a significant role (F_{2; 2.807}=6.947; p=0.082). In conclusion, average premature saccade latency was not affected by ketamine.

### Example 2: Reduction of early saccades occurrence

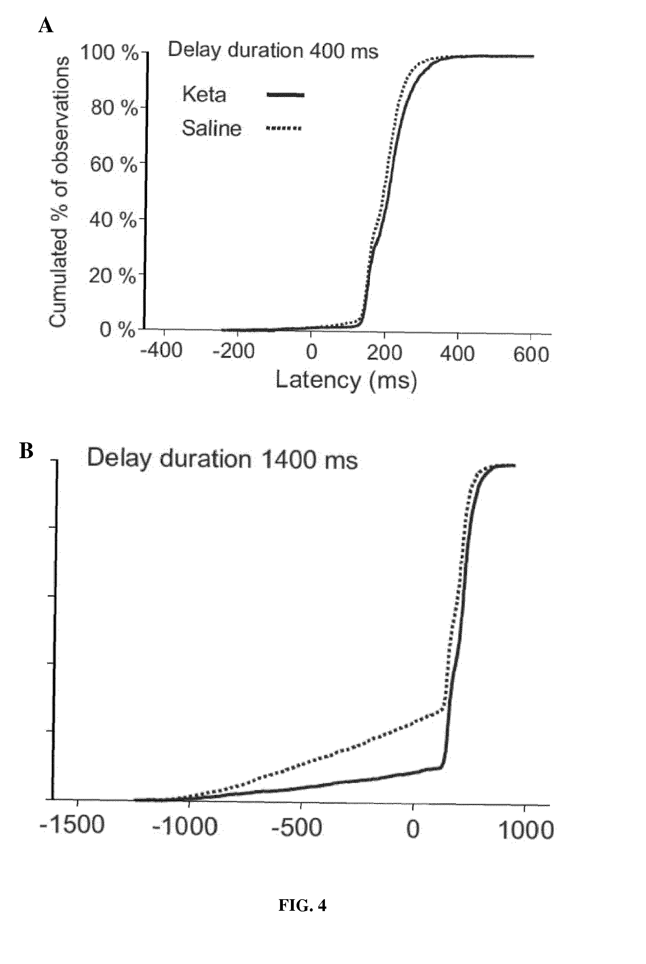

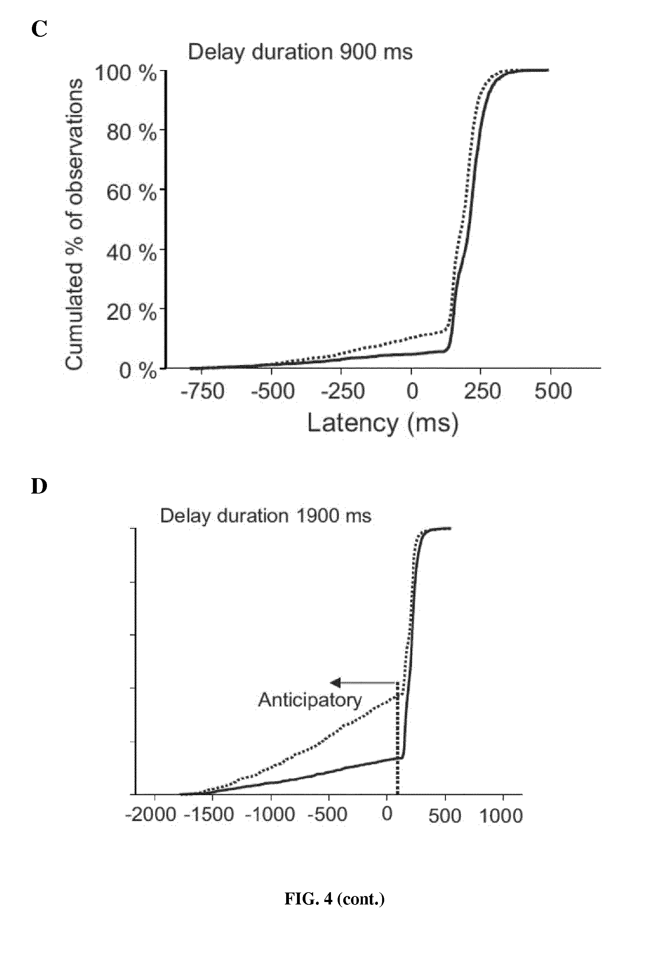

The result of the analysis on average latencies seems contradictory. Indeed, the significant interaction effect of ketamine and delay duration on saccadic latencies vanished when anticipatory and visually-guided saccades were analyzed separately. In order to further investigate this result, cumulative latency distributions of all saccades for the different delay durations were computed. **Figure 4** shows the cumulative distributions for the four delay durations tested (group data; controls, ketamine). It can be observed that cumulative latency distributions are composed of an early part (before 100 ms on the X-axis) representing premature saccades and a later part with a steeper slope representing visually-guided saccades. It can be observed that the number of premature saccades was strongly and significantly reduced after ketamine injection (compare solid and dashed curves; Pearson chi-square test = 437.496; p=0.000; see **Table 2** for group data and **Table 3** for subject-by-subject percentages).

### Product * Premature saccades Crosstabulation

**Table 2.**

| | | Premature | | |
|---|---|---|---|---|
| | | 0 | 1 | Total |
| Produit | 1 | 6996 | 579 | 7575 |
| | 0 | 5250 | 1279 | 6529 |
| Total | | 12246 | 1858 | 14104 |

**Table 3.**

| | Saline | | | Ketamine | | |
|---|---|---|---|---|---|---|
| Monkey | Visually guided | Premature | Total | Visually guided | Premature | Total |
| L | 1367 (65%) | 719 (35%) | 2086 | 1520 (79% | 408 (21%) | 1928 |
| S | 1697 (97%) | 52 (3%) | 1749 | 2939 (93%) | 21 (7%) | 2960 |
| Y | 2186 (81%) | 508 (19%) | 2694 | 2537 (94%) | 150 (6%) | 2687 |
| Total | 5250 | 1279 | 6529 | 6996 | 579 | 7575 |

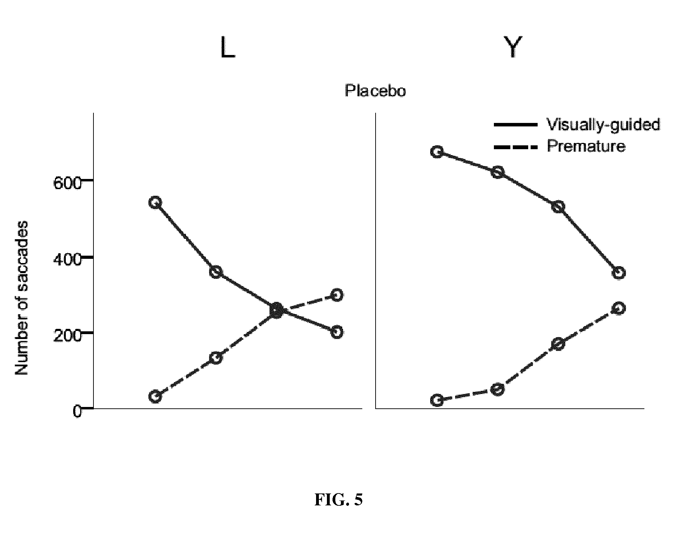

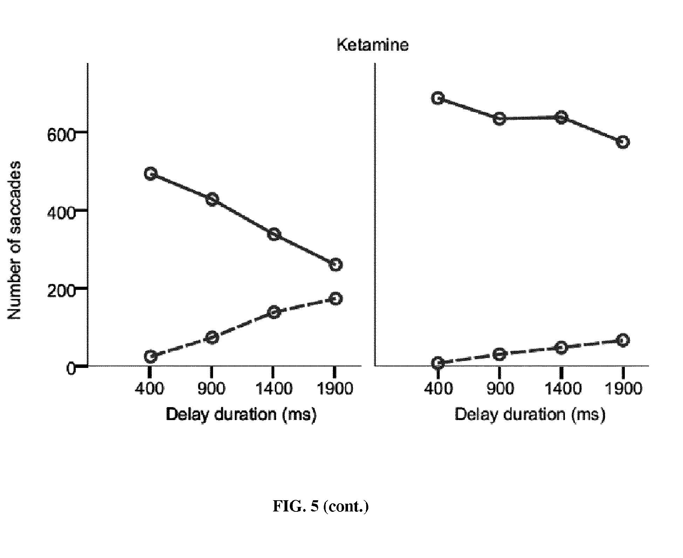

This reduction was particularly strong for long delay durations (1400 ms and 1900 ms). Indeed, during long delays, a longer period of inhibition of a reflexive saccade to the eccentric box was required, and 'false alarms' (premature saccades) increased. This is future exemplified on **Figure 5** shows that shows the evolution of the number of visually-guided (solid curves) and premature saccades (dashed curves) as a function of delay duration for the 2 monkeys of this study for whom a large number of premature saccades were recorded (left column monkey L; right column monkey Y). In the placebo condition in both monkeys (upper two figures), the number of rewarded visually-guided saccades clearly decreased with increasing delay duration. Given that a saccade is either premature or visually guided, unless monkeys aborted the trial by looking away from the fixation target, the number of anticipatory saccades increased with delay duration. In monkey L, there were even more premature than visually guided saccades for the 1900 ms delay. Given that premature saccades were not rewarded, they could be considered as inappropriate impulsive responses triggered by the urge to move during long delays. After ketamine injection, the number of visually-guided saccades decreased more slowly with increasing delay duration and consequently the number of premature saccades increased more slowly.

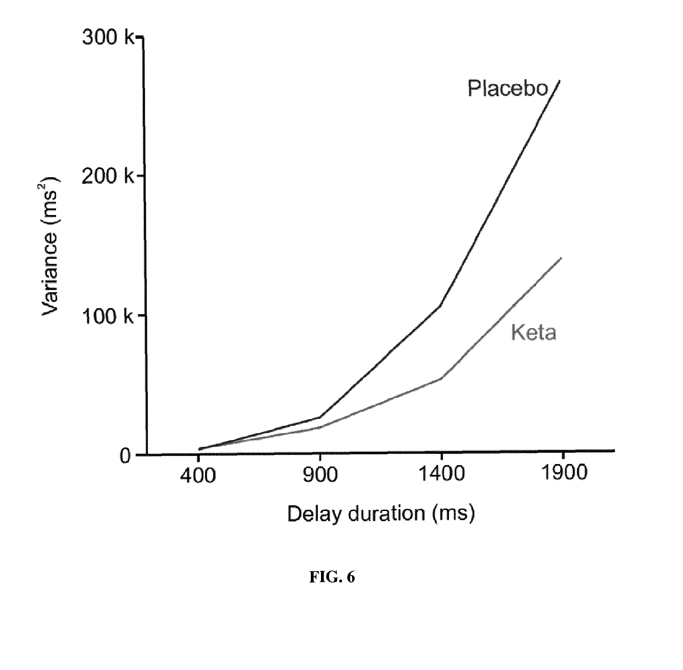

Cumulative latency distributions show that ketamine injection did not simply increase the latency of anticipatory saccades on average but reduced their occurrence. As a result, latency variance decreased. **Figure 6** shows the effect of ketamine injection (grey curve) on latency variance. A reduction of global variance in response suggests that the small doses of ketamine used in the present study increased performance in the present task.

### Example 3: Time course of observed effects

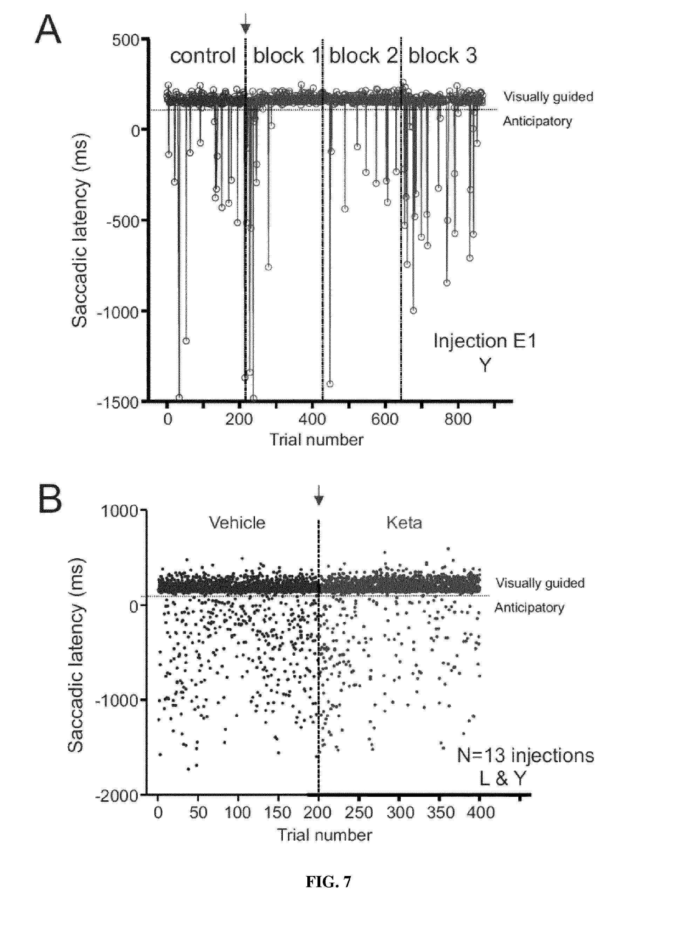

The suppression of anticipatory saccades manifested itself very rapidly. **Figure 7A** shows a series of trials before and after a single ketamine injection in monkey Y (*arrow*). Approximately 100 trials after ketamine injection, the number of early anticipatory saccades was strongly reduced. During the second block post-injection, the occurrence of anticipatory saccades re-increased progressively. Only during the third block following injection did premature saccades frequency returned back to control levels. A similar effect was observed in monkey L. **Figure 7B** shows saccadic latency as a function of trial number for all 13 ketamine injections in monkeys Y&L (too few premature saccades were recorded in monkey S). The first block of 200 control trials *(black dots)* is represented followed by the first post-injection blocks of 200 trials (*grey dots after vertical line*). It can be observed that the density of points representing of early latencies decreased after injection without completely suppressing premature saccade occurrence. The same phenomenon was found during all injections in both monkeys.

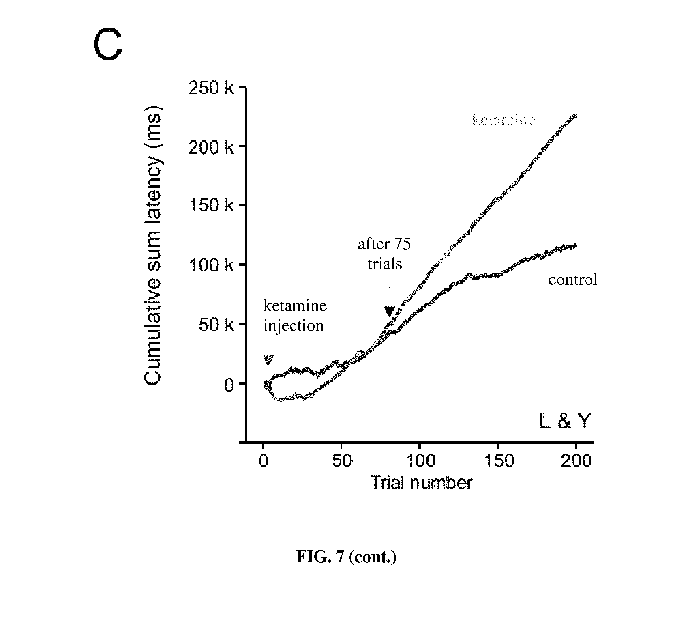

In order to more precisely determine how long it took for ketamine to start modifying saccade latency, the cumulated sum of latencies is represented on **Figure 7C****.** The ordinate represents the cumulated sum of latencies and does not have any physical meaning. It increases more rapidly if more positive values of saccadic latency are observed. Given that visually guided saccades were always more numerous, the cumulated sum increased for both control (*black curve*) and ketamine (*grey curve*) conditions. However, after approximately 75 trials, the two cumulated sums diverged. As a single trial lasted 3300 ms on average, it took approximately 4 minutes after the onset of the second block of trials for the injection for ketamine to affect saccade latency.

### Example 4: Other effects

It could be suggested that saccades during ketamine injection experiments were abnormal. However, ketamine injection did not reduce saccade amplitude in group data (monkeys L and Y). An ANOVA with injected product (ketamine or vehicle) as fixed-factor (n=5137 saccades) and monkey as random factor did not show a significant main effect of ketamine [F(1,1)=0.003; p=0.963] or monkey identity [F(1,1)=1.204; p=0.471]. On average in group data amplitude was not affected. However, a significant interaction between product and monkey identity was found [F(1,5169)=41.421; p=0.000]. This effect was due to a different influence of ketamine on saccade amplitude. Indeed, saccade amplitude was *reduced* by ketamine in monkey L (control: 5.7±0.05 deg; 5.2±0.05 deg; n=4014) and *increased* in monkey Y (control: 5.7±0.03 deg; 6.2±0.04 deg; n=1159). Note that all amplitude changes amounted to only 10 % approximately.

The dose of ketamine used in the present study (0.25 mg/kg) did not evoke spontaneous nystagmus or centripetal drift of the eye as could be observed with higher doses (e.g. 0.6 mg/kg; see Condy et al. Biol Psychiatry 2005; 57: 366-372). Therefore, sedation is a very unlikely hypothesis to explain observed results. However, in order to further test the potential influence of a light sedation in the present results, we compared the proportion of valid trials with and without ketamine. During valid trials, monkeys made a saccade towards the eccentric target (premature or visually-guided). However, a trial could be aborted if the animal did not foveate the initial fixation target rapidly enough, or moved away from the eccentric target when it appeared or simply failed to react to the appearance of the fixation point (trial omission). It could be suggested that light sedation could increase the proportion of aborted trials. However, Figure 8 shows that after ketamine injection, the proportion of valid trials decreased by only ∼5 % in monkey L, increased by ∼10 % in monkey S, and was unchanged in monkey Y. At the group level, ketamine did not statistically alter the proportion of valid trials [F_{(1,22)}=0.00; P=0.99; df=23].

In conclusion, the effect of ketamine injection on premature (=anticipatory) saccades reduction was consistent in the 2 subjects who generate anticipatory saccades frequently. Ketamine modestly reduced saccadic amplitude. This small amplitude reduction cannot explain the reduction of anticipatory saccades observed. Moreover, reduction of anticipatory saccades cannot be attributed to light sedation.

Consequently, anticipatory saccades occurrence could be reduced by ketamine.

We suggest that beneficial effects of low doses of ketamine on impulsivity will be found in all tasks where there is a challenging competition between alternative goals, as it is often the case in real-life situations. Treating impulsivity is an important intervention strategy in psychiatry. Impulsivity scores based on questionnaires should be compared with premature saccades occurrence in human subjects with low doses of ketamine or placebo i.m. We suggest that the frequency of occurrence of premature saccades might be a particularly useful tool in impulsivity evaluation and study in primates and in a clinical environment.

### Materials and Methods

Three monkeys participated in the present study (referred to as L, S, Y).

### Apparatus

Subjects sat in darkness, facing a CRT screen, which presented stimuli at a frequency of 60 Hz. An EyeLink 1000 infrared eye tracking system (SR Research, Mississauga, Ontario) was used to record movements of the right eye at 1 KHz. All experiments were run with homemade stimulus generation software based on a real time Linux kernel (Xenomaï). Stimulus display and oculomotor data collection were synchronized on a frame-by-frame basis. Saccades were detected offline in MATLAB (MathWorks, Natic, MA) with a velocity threshold of 30 deg/sec.

Each trial started with an initial fixation period (referred to as 'initial fixation'), with a small cross (0.7 deg) appearing on the CRT screen for a random duration (850±100 ms; see 'X' on Fig. 1). At the end of this random delay, two empty square 'boxes' appeared on the screen (1.4x1.4 deg), one in the center of the screen and one 9 deg eccentric, randomly to the right or to the left. The empty boxes were displayed to provide upcoming target position beforehand. Afterwards, a square target (1.4x1.4 deg) was flashed in the central box for 50 ms. Extinction of the initial target indicated to subjects the beginning of the delay period. Subjects were required to hold on fixation of the central box until another target (1.4x1.4 deg) was briefly presented for 50 ms in the eccentric box. In the variable fore period design, the delay period could take one of 4 different values with the same probability: 400 ms, 900 ms, 1400 ms and 1900 ms. In order to receive reward, subjects had to maintain fixation of the central box and wait for the appearance of the eccentric target. This required a strong inhibition of early saccades before the appearance of the eccentric target. Early saccades were not rewarded. This paradigm was designed to test inhibition during the delay period.

Each experimental session started with a block of 200 trials before injection to measure the baseline saccadic latency on a day-by-day basis. On a randomly selected day ketamine (0.250 mg/kg) was injected i.m. or a placebo saline solution (NaCl 0.9 %) of the same volume (0.3 ml). Only saccadic latencies less than 600 ms were considered for further analysis.

### Statistical analyses

All analyses were performed using the univariate General Linear Model analysis of variance (ANOVA) in SPSS (SPSS, International Business Machines, Armonk USA). The significance threshold α for all analysis was 0.05. Monkey identity was used as a random factor to take into account the influence of uncontrolled variability observed between subjects. Fixed factors will be given in the text for each analysis. The dependent variable measured was saccadic latency or amplitude.

## Claims

1. A composition for use in the treatment of a mental disorder with occurrence of aggressive and/or impulsive behavior in a subject in need thereof, comprising a sub-anesthetic dose of a compound antagonizing NMDA receptors.

2. The composition for use according to claim **1,** wherein said compound is a noncompetitive or an uncompetitive antagonist of the NMDA receptor.

3. The composition for use according to anyone of claims **1** to **2,** wherein said compound is ketamine, amantadine, tiletamine, phencyclidine (PCP), dizocilpine MK-801, Argiotoxin 636, dextrorphan, HU-211, Rhynchophylline, memantine, aptiganel, ifenprodil, lanicemine, Remacemide and/or analog and/or functional derivatives thereof.

4. The composition for use according to anyone of claims **1** to **3,** wherein said compound is ketamine and/or an analog and/or a functional derivative thereof.

5. The composition for use according to anyone of claims **1** to **3,** wherein said compound is memantine and/or an analog and/or a functional derivative thereof.

6. The composition for use according to anyone of claims **1** to **5,** further comprising at least one pharmaceutical excipient.

7. The composition for use according to anyone of claims **1** to **6,** wherein said composition is to be administered in a subject in need thereof by topical, transdermal, intramuscular, subcutaneous, oral, parenteral, intranasal administration.

8. The composition for use according to anyone of claims **1** to **7,** wherein said composition is provided in a sustained-release form.

9. The composition for use according to anyone of claims **1** to **8,** wherein said composition is formulated as a patch.

10. The composition for use according to anyone of claims **1** to **9,** wherein said disorder comprises: personality disorders, anxiety disorders, mood disorders, impulse control and addiction disorders, attention deficit hyperactivity disorder (ADHD), obsessive-compulsive disorder (OCD), Tourette syndrome, Impulse control disorder (ICD), Blepharospasm, Tic disorder, Dissocial personality disorder, Intermittent explosive disorder (IED), Bipolar disorder, Pyromania, Kleptomania, paranoid personality disorders, schizoid personality disorders, schizotypal personality disorders, social anxiety disorder, panic disorder, agoraphobia, post-traumatic stress disorder, antisocial personality disorders, borderline personality disorders, histrionic personality disorders, narcissistic personality disorders.

11. A patch for use in the treatment of a mental disorder with occurrence of aggressive and/or impulsive behavior, comprising the composition according to anyone of claims **1** to **10,** and an acceptable carrier.
